# EUROPÄISCHE PATENTSCHRIFT

(11) **EP 0 556 672 B1**
(45) Veröffentlichungstag und Bekanntmachung des Hinweises auf die Patenterteilung: **06.08.1997**
(21) Anmeldenummer: 93101840.2
(22) Anmeldetag: 05.02.1993
(51) Int. Cl.: C07D 295/155, C07C 279/22, A61K 31/155

(54) **Aminosubstituierte Benzoylguanidine mit antiarrhythmischen Eigenschaften**
Amino-substituted benzoylguanidines with antiarrhythmic properties
Benzoylguanidines amino-substitués possédant des propriétés antiarythmiques

(30) Priorität: 15.02.1992 DE 4204577
(43) Veröffentlichungstag der Anmeldung: 25.08.1993
(73) Patentinhaber: HOECHST AKTIENGESELLSCHAFT, 65926 Frankfurt am Main (DE)
(72) Erfinder: Englert, Heinrich, Dr., W-6238 Hofheim/Ts (DE); Mania, Dieter, Dr., W-6240 Königstein/Ts (DE); Lang, Hans-Jochen, Dr., W-6238 Hofheim/Ts (DE); Scholz, Wolfgang, Dr., W-6236 Eschborn (DE); Linz, Wolfgang, Dr., W-6500 Mainz (DE); Albus, Udo, Dr., W-6364 Florstadt (DE)

(56) Entgegenhaltungen:
- EP-A- 0 416 499
- US-A- 3 780 027
- BULLETIN DE LA SOCIETE CHIMIQUE DE FRANCE, 1964, Paris, FR, Seiten 1517 - 1524 E. RIESZ, ET AL.: 'Constitution chimique et affinité pour les composés macromoléculaires. I. Recherches sur les dérivés benzoylés de la guanidine'

## Beschreibung

Die Erfindung betrifft Benzoylguanidine der Formel I 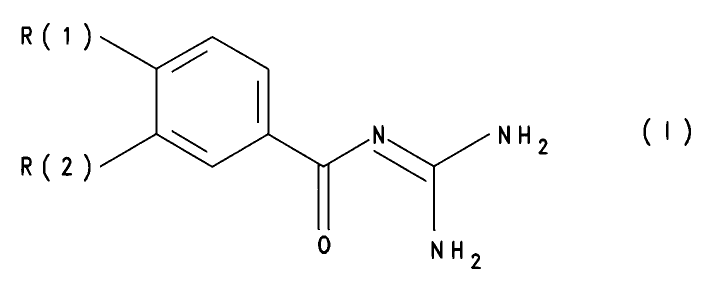 worin bedeuten:
- R(1) oder R(2): eine Aminogruppe -NR(3)R(4),
mit R(3) und R(4) - gleich oder verschieden - H, C₁-C₆-Alkyl, C₃-C₇-Cycloalkyl, oder
R(3) gleich Phenyl-(CH₂)ₚ- mit p gleich 0, 1, 2, 3 oder 4, oder Phenyl,
wobei Phenyl jeweils unsubstituiert ist oder einen bis zwei Substituenten aus den Gruppen Fluor, Chlor, Methyl oder Methoxy trägt,
- R(3) und R(4): auch gemeinsam eine geradkettige oder verzweigte C₄-C₇-Methylenkette sein können, wobei ein -CH₂-Glied der Methylenkette durch Sauerstoff, S oder NR(5) ersetzt sein kann und R(5) für H oder niedriges Alkyl steht,
der jeweils andere Substituent R(1) oder R(2) F, Cl, C₁-C₄-Alkyl, C₁-C₄-Alkoxy,
- CF₃, CₘF₂ₘ₊₁-CH₂-,: Benzyl oder Phenoxy,
wobei der jeweilige Phenylrest unsubstituiert ist oder einen bis zwei Substituenten in der Bedeutung von Methyl, Methoxy, Fluor oder Chlor trägt,
und worin m gleich 1, 2 oder 3 ist,
sowie deren pharmazeutisch verträgliche Salze.

Enthält einer der Substituenten R(1) bis R(4) ein Asymmetriezentrum, so gehören sowohl S als auch R konfigurierte Verbindungen zur Erfindung. Die Verbindungen können als optische Isomere, als Diastereoisomere, als Racemate oder als Gemische derselben vorliegen.

Die bezeichneten Alkylreste können sowohl geradkettig wie verzweigt vorliegen.

Bevorzugt sind Verbindungen der Formel I, worin bedeuten:
einer der beiden Substituenten R(1) oder R(2) eine Aminogruppe -NR(3)R(4),
   worin R(3) und R(4) gemeinsam eine Methylenkette (CH₂)ₙ mit n = 4 - 5 sind
und der jeweilige andere Substituent R(1) oder R(2) Chlor oder Phenoxy.

Besonders bevorzugt sind 4-Chlor-3-(1-pyrrolidino)-benzoyl-guanidin, 4-Methyl-3-(1-pyrrolidino)-benzoyl-guanidin, 4-Chlor-3-(1-piperidino)-benzoyl-guanidin, 4-Methyl-3-(1-piperidino)-benzoyl-guanidin, 4-Phenoxy-3-(1-pyrrolidino)-benzoyl-guanidin sowie 4-(2-Chlorphenoxy)-3-(1-pyrrolidino)-benzoyl-guanidin und deren pharmakologisch verträgliche Salze.

Die Verbindungen I sind substituierte Acylguanidine.

Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 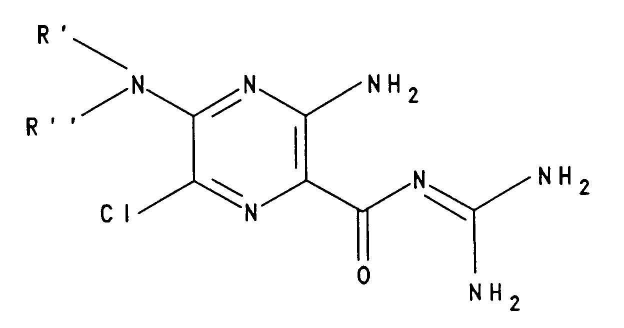
Amilorid: R', R'' = H
Dimethylamilorid: R', R'' = CH₃
Ethylisopropylamilorid: R' = C₂H₅, R'' = CH(CH₃)₂

Darüber hinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

Im US-Patent 3 780 027 werden Acylguanidine beansprucht, die strukturell den Verbindungen der Formel I ähnlich sind. Der entscheidende Unterschied zu den erfindungsgemäßen Verbindungen I besteht darin, daß es sich um trisubstituierte Benzoylguanidine handelt, die sich in ihrem Substitutionsmuster von im Handel befindlichen Diuretika, wie Bumetanid und Furosemid, ableiten und eine für die angestrebte salidiuretische Wirkung wichtige Aminogruppe in Position 2 bzw. 3 zur Carbonylguanidingruppe tragen. Entsprechend wird für diese Verbindungen eine starke salidiuretische Wirksamkeit berichtet.

In EP 91416499 (EP-A-416 499) werden Benzoylguanidine beansprucht, die in der dem Rest R(2) entsprechenden Position SOₙ-Reste tragen. Sie haben nur noch antiarrhythmische Wirkungen.

Aus Bull.Soc. Chim. Fr. [1964], Seite 1517 - 1524, besonders Seite 1523, Absatz IV ist die Verbindung 4-Amino-benzoylguanidin als Vorprodukt für die Herstellung von Diazofarbstoffen bekannt. Angaben zur Wirsamkeit dieser Verbindung auf die Hemmung des Na⁺/H⁺-Austauschs sind nicht gemacht.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoff-Mangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindung auch als Arzneimittel zur Behandlung von Ischämien des Nervensystems, insbesondere des Zentralnervensystems, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na+/H+ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindung der Formel I für die präventive Therapie zur Verhinderung der Genese des Bluthochdrucks, beispielweise der essentiellen Hypertonie, geeignet.

Die Erfindung betrifft weiterhin ein Verfahren zur Herstellung der Verbindungen I, dadurch gekennzeichnet, daß man
Verbindungen der Formel II 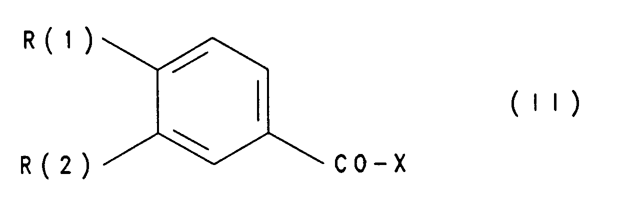 mit Guanidin der Formel III 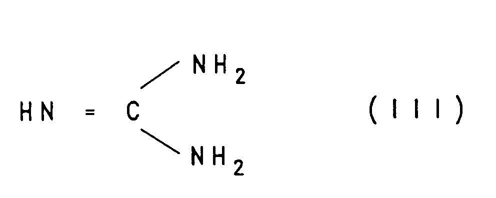 umsetzt, worin R(1) und R(2) die angegebene Bedeutung besitzen und X für eine leicht nucleophil substituierbare leaving group steht.

Die aktivierten Säurederivate der Formel II, worin X eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, X = Cl), die man ihrerseits wiederum in an sich bekannter Weise aus den zugrundeliegenden Carbonsäuren (Formel II, X = OH) beispielsweise mit Thionylchlorid herstellen kann.

Neben den Carbonsäurechloriden der Formel II (X = Cl) lassen sich auch weitere aktivierte Säurederivate der Formel II in an sich bekannter Weise direkt aus den zugrundeliegenden Benzoesäure-Derivaten (Formel II, X = OH) herstellen, wie beispielsweise die Methylester der Formel II mit X = OCH₃ durch Behandeln mit gasförmigem HCl in Methanol, die Imidazolide der Formel II durch Behandeln mit Carbonyldiimidazol (X = 1-Imidazolyl, Staab, Angew. Chem. Int. Ed. Engl. 1, 351 - 367 (1962)), die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC). Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin der Formel III erfolgt in an sich bekannter Weise in einem protischen oder aprotischen polaren aber inerten organischen Lösungsmittel. Dabei haben sich bei der Umsetzung der Benzoesäuremethylester (II, X = OMe) mit Guanidin in Methanol oder THF zwischen 20 °C und Siedetemperatur dieser Lösungsmittel bewährt. Bei den meisten Umsetzungen von Verbindungen II mit Guanidinen III als freie Basen wurde vorteilhaft in aprotischen inerten Lösungsmitteln wie THF, Dimethoxyethan, Dioxan gearbeitet. Aber auch Wasser kann als Lösungsmittel bei der Umsetzung von II und III verwendet werden.

Wenn X = Chlor bedeutet, arbeitet man vorteilhaft unter Zusatz eines Säurefängers, z. B. in Form von überschüssigem Guanidin zur Abbindung der Halogenwasserstoffsäure.

Ein Teil der zugrundeliegenden Benzoesäurederivate der Formel II und das verwendete Guanidin der Formel III sind bekannt und in der Literatur beschrieben. Die unbekannten Verbindungen der Formel II können nach literaturbekannten Methoden hergestellt werden, indem man beispielsweise einen Benzoesäuremethylester II mit R(1) und R(2) in der eingangs erwähnten Bedeutung, speziell mit R(3), R(4) gleich H, sowie X beispielsweise gleich OCH_{3,} mit einem Dicarbonsäureanhydrid wie beispielsweise Glutarsäureanhydrid oder Bernsteinsäureanhydrid in eine Verbindung der Formel IV überführt, worin R(1) oder R(2) nun ein cyclisches Imid der Formel IVa bedeuten mit n in der Methylenkette gleich 3 bis 7.

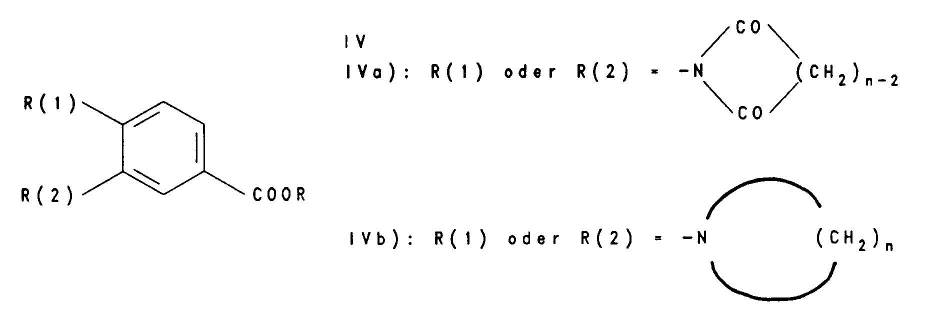

Dieses Imid IVa wird sodann durch Einwirkung von Natriumboranat und Bortrifluoridetherat in den entsprechenden Benzoesäuremethylester der Formel IVb übergeführt. Dieser kann direkt weiter mit Guanidin umgesetzt werden zu Verbindungen der Formel I.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.
Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg, vorzugsweise 0,01 mg bis 10 mg, vorzugsweise 1 mg. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i. v. Anwendung, etwa bei einem Infarktpatienten auf der Intensivstation können bis zu 100 mg pro Tag notwendig werden.

### Experimenteller Teil

### Allgemeine Reaktionsvorschrift (Vorschrift A) zur Umsetzung der Verbindungen der Formel II mit X in der Bedeutung OCH₃ zu Benzoylguanidin Hydrochloriden I,

### Methode A:

0.02 Mol des Methylesters II werden in 50 ml Methanol gelöst und unter Schutzgasatmosphäre (Argon) mit 3.6 g Guanidin (freie Base) versetzt. Man kocht 12 h unter Rückfluß, entfernt das Lösemittel durch Eindampfen im Vakuum und nimmt den Rückstand in Wasser auf. Extraktion mit Methylenchlorid, Eindampfen des Lösemittels liefert einen öligen Rückstand, der nach Lösen in methanolischer HCl durch Zugabe von Ether erneut abgeschieden wird. Das so erhaltene Öl wird durch Chromatographie an Kieselgel (Laufmittel: Methylenchlorid/Methanol 20 : 1) gereinigt.

### Allgemeine Vorschrift (Vorschrift B) zur Herstellung von Benzoylguanidinen (I) aus Benzoesäuren (II, X = OH)

0,01 M des Benzoesäurederivates der allgemeinen Formel II (X = OH) löst bzw. suspendiert man in 60 ml wasserfreien THF und versetzt sodann mit 1,78 g (0,011 M) Carbonyl-diimidazol. Nach dem Rühren über 2 Stunden bei Zimmertemperatur werden 2,95 g (0,05 M) Guanidin in die Reaktionslösung eingetragen. Nach Rühren über Nacht destilliert man das THF unter vermindertem Druck (Rotavapor) ab, versetzt mit Wasser, stellt mit 2N Salzsäure auf pH 6 - 7 und filtriert das entsprechende Benzoylguanidin-Derivat (Formel I) ab.

Die so erhaltenen Benzoylguanidine können durch Behandeln mit wäßriger oder methanolischer Salzsäure oder anderen pharmakologisch verträglichen Säuren in die entsprechenden Salze übergeführt werden.

Das cyclische Imid IVa (0.05 M) wird in 50 ml Diglyme gelöst und mit 14,1 ml (0,1144 M) BF₃·Et₂O versetzt. Nach Abkühlen auf 0 - 5 °C werden 4,3 g (0,1144 M) NaBH₄ bei max. 5 °C zugegeben und die Mischung 6 h bei unter 5 °C gerührt. Anschließend wird auf Eis-H₂O gegossen und das sich abscheidende Öl durch Abdekantieren abgetrennt. Aufnehmen in Essigsäureethylester, Trocknen mit MgSO₄ und Abdampfen des Lösemittels liefert das gewünschte Produkt, das im allgemeinen ohne weitere Reinigung zum Acylguanidin I weiter umgesetzt werden kann.

### Allgemeine Vorschrift zur Herstellung von cyclischen Imiden IVa aus Aminen IVb

0.1 Mol des Amins der Formel IVb sowie 0,3 Mol eines Dicarbonsäureanhydrids wie etwa Bernsteinsäureanhydrid oder Glutarsäureanhydrid werden bei 180°C zusammengeschnolzen und bei dieser Temperatur 10 h gebraten. Danach wird in Methanol aufgenommen und in Wasser eingerührt, Das auskristallisierte Produkt abgesaugt und aus Ethanol umkristallisiert.

### Beispiel 1:

### 4-Phenoxy-3-N-pyrrolidinobenzoylguanidin hydrochlorid

Aus 4-Phenoxy-3-N-pyrrolidinobenzoesäuremethylester und Guanidin.
   Schmp.: 225 - 227 °C; MS: M(+) = 324 (ber. Mol.G. = 324.37), Vorschrift A.

### Beispiel 2:

### 4-Methyl-3-N-pyrrolidinobenzoylguanidin hydrochlorid

Aus 4-Methyl-3-N-pyrrolidinobenzoylbenzoesäuremethylester und Guanidin (Vorschrift A).
   Schmp.: 235 - 238 °C; MS: M(+) = 226 (ber. Mol.G. = 226.67)

### Beispiel 3:

### 4-Chlor-3-N-ethylaminobenzoylguanidin hydrochlorid

Aus 4-Chlor-3-N-ethylaminobenzoesäuremethylester und Guanidin (Vorschrift A).
   Schmp.: 196 - 198 °C; MS: M(+) = 240 (ber. Mol.G. = 240.69)

### Beispiel 4:

### 4-Phenoxy-3-N-propylaminoguanidin hydrochlorid

Aus 4-Phenoxy-3-N-propylaminobenzoesäuremethylester und Guanidin (Vorschrift A).
   Schmp.: 221 - 223 °C; MS: M(+) = 312 (ber. Mol.G. = 312.36)

### Beispiel 5:

### 4-Chlor-3-N-pyrrolidinobenzoylguanidin hydrochlorid

Schmp.: 268 - 270 °C; MS: M(+) = 358 (ber. Mol.G. = 266.73)
   Aus 4-Chlor-3-N-pyrrolidinobenzoesäuremethylester und Guanidin (Vorschrift A).

### Beispiel 6:

### 4-Methoxy-3-N-propylaminoguanidin hydrochlorid

Aus 4-Methoxy-3-N-propylaminobenzoesäuremethylester und Guanidin (Vorschrift A).
   Schmp.: 231 - 233 °C; MS: M(+) = 250 (ber. Mol.G. = 250.30)

### Beispiel 7:

### 4-Chlor-3-N-benzylaminoguanidin hydrochlorid

Aus 4-Chlor-3-N-benzylaminobenzoesäuremethylester und Guanidin (Vorschrift A).
   Schmp.: 91 - 93 °C; MS: M(+) = 302 (ber. Mol.G. = 302.76)

### Beispiel 8:

### 4-(2-Chlorphenoxy)-3-N-pyrrolidinobenzoylguanidin hydrochlorid

Aus 4-(2-Chlorphenoxy)-3-N-pyrrolidinobenzoesäuremethylester und Guanidin (Vorschrift A).
   Schmp.: 121 °C; MS: M(+) = 358 (ber. Mol.G. = 358.82)

### Beispiel 9:

### 4-(2,3-Dichlorphenoxy)-3-N-pyrrolidinobenzoylguanidin hydrochlorid

Aus 4-(2,3-Dichlorphenoxy)-3-N-pyrrolidinobenzoesäuremethylester und Guanidin (Vorschrift A).
   Schmp.: 169 - 170 °C; MS: M(+) = 391 (ber. Mol.G. = 393.27)

### Beispiel 10:

### 4-(2-Methylphenoxy)-3-N-pyrrolidinobenzoylguanidin hydrochlorid

Aus 4-(2-Methylphenoxy)-3-N-pyrrolidinobenzoesäuremethylester und Guanidin (Vorschrift A).
   Schmp.: 185 - 200 °C; MS: M(+) = 338 (ber. Mol.G. = 338.4)

### Beispiel 11:

### 4-(4-Chlorphenoxy)-3-N-pyrrolidinobenzoylguanidin hydrochlorid

Aus 4-(4-Chlorphenoxy)-3-N-pyrrolidinobenzoesäuremethylester und Guanidin (Vorschrift A).
   Schmp.: 120 - 135 °C; MS: M(+) = 358. (ber. Mol.G. = 358.82)

### Beispiel 12:

### 4-(2-Methoxyphenoxy)-3-N-pyrrolidinobenzoylguanidin hydrochlorid

Aus 4-(2-Methoxyphenoxy)-3-N-pyrrolidinobenzoesäuremethylester und Guanidin (Vorschrift A).
   Schmp.: 180 - 195 °C; MS: M(+) = 354 (ber. Mol.G. = 354.4)

### Beispiel 13:

### 4-Chlor-3-N-piperidinobenzoylguanidin hydrochlorid

Aus 4-Chlor-3-N-piperidinobenzoesäuremethylester und Guanidin (Vorschrift A).
   Schmp.: 210 - 212 °C; MS: M(+) = 280 (ber. Mol.G. = 280.76)

### Beispiel 14:

### 4-Methyl-3-N-piperidinobenzoylguanidin hydrochlorid

Aus 4-Methyl-3-N-piperidinobenzoesäuremethylester und Guanidin (Vorschrift A).
   Schmp.: 160 - 162 °C; MS: M(+) = 260 (ber. Mol.G. = 260.33)

### Beispiel 15:

### 4-Chlor-3-N-pentylaminobenzoylguanidin hydrochlorid

Aus 4-Chlor-3-N-pentylaminobenzoesäuremethylester und Guanidin (Vorschrift A).
   Schmp.: 80 - 82 °C; MS: M(+) = 282 (ber. Mol.G. = 282.77)

### Beispiel 16

4-Dimethylamino-benzoylguanidin-hydrochlorid
   aus 4-Dimethylamino-benzoesäure und Guanidin (Vorschrift B), farblose Kristalle, Fp. > 285 °C.

### Beispiel 17

### 4-Piperidino-benzoylguanidin-hydrochlorid

aus 4-Piperidino-benzoesäure und Guanidin (Vorschrift B), farblose Kristalle, Fp. 189 °C,

### Beispiel 18

### 3-Chlor-4-piperidino-benzoylguanidin-hydrochlorid

aus 3-Chlor-4-piperidino-benzoesäure und Guanidin (Vorschrift B), farblose Kristalle, Fp. 155 °C,

### Beispiel 19

### 4-N-Methyl-N-(2-phenylethyl)amino-benzoylguanidin-acetat

aus 4-N-Methyl-N-(2-phenylethyl)amino-benzoesäure und Guanidin (Vorschrift B), farblose Kristalle, Fp. 173 °C.

### Pharmakologische Daten

### Narkotisierte Ratte mit Coronarligatur

### a) Methode

Männliche Sprague Dawley Ratten wurden mit Thiopental-natrium in einer Dosis von 100 mg/kg i. p. narkotisiert. Nach Öffnen und Spreizen des Brustraumes wurde ein Zugang zur linken Coronararterie geschaffen und dort mittels einer atraumatischen Nadel ein Seidenstreifen um die linke Coronararterie eingebracht. Nach einer Äquilibrierungsperiode von 10 min wurde die Substanz i. v. verabreicht und 5 min später die Coronararterie mittels des Seidenstreifens verschlossen. Ventrikuläre Extrasystolen, ventrikuläre Tachyarrhythmien sowie Kammerflimmern wurden nach den Richtlinien der Lambeth Konvention (London, 1987) bestimmt. Die Substanzen wurden in DMSO/Kochsalzlösung verabreicht, wobei eine solche Lösung 1 Volumenprozent DMSO enthält. In Kontrollexperimenten wurden die Tiere mit dem Lösungsmittel alleine behandelt. Das applizierte Lösungsmittelvolumen betrug in allen Fällen 1 ml/kg.

### b) Ergebnisse:

Die Substanz aus Beispiel 8 wurde in einer Dosis von 1 mg/kg verabreicht:
Dauer der ventrikulären Tachyarrhythmien: 4 ± 2 sec (n = 6)
Bei unbehandelten Kontrolltieren erhält man: 41 ± 19 sec (n = 4)

## Patentansprüche

1. Benzoylguanidine der Formel I 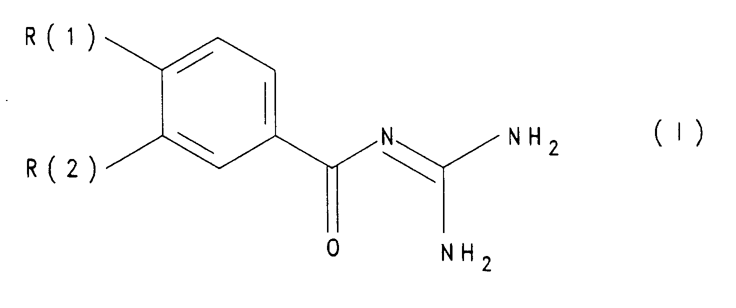 worin bedeuten:
R(1) oder R(2) eine Aminogruppe -NR(3)R(4),
mit R(3) und R(4) - gleich oder verschieden - H, C₁-C₆-Alkyl, C₃-C₇-Cycloalkyl, oder
R(3) gleich Phenyl-(CH₂)ₚ- mit p gleich 0, 1, 2, 3 oder 4, oder Phenyl,
wobei Phenyl jeweils unsubstituiert ist oder einen bis zwei Substituenten aus den Gruppen Fluor, Chlor, Methyl oder Methoxy trägt,
R(3) und R(4) auch gemeinsam eine geradkettige oder verzweigte C₄-C₇-Methylenkette sein können, wobei ein -CH₂-Glied der Methylenkette durch Sauerstoff, S oder NR(5) ersetzt sein kann und R(5) für H oder niedriges Alkyl steht,
der jeweils andere Substituent R(1) oder R(2) F, Cl, C₁-C₄-Alkyl, C₁-C₄-Alkoxy, CF₃, CₘF₂ₘ₊₁-CH₂-, Benzyl oder Phenoxy,
wobei der jeweilige Phenylrest unsubstituiert ist oder einen bis zwei Substituenten in der Bedeutung von Methyl, Methoxy, Fluor oder Chlor trägt,
und wobei m gleich 1, 2 oder 3 ist,
sowie deren pharmazeutisch verträgliche Salze.

2. Benzoylguanidine der Formel I nach Anspruch 1, dadurch gekennzeichnet, daß darin bedeuten:
einer der beiden Substituenten R(1) oder R(2) eine Aminogruppe -NR(3)R(4), worin R(3) und R(4) gemeinsam eine Methylenkette (CH₂)ₙ mit n = 4 - 5 sind und der jeweilige andere Substituent R(1) oder R(2) Chlor oder Phenoxy.

3. Benzoylguanidine der Formel I nach Anspruch 1 dadurch gekennzeichnet, daß sie sind:
4-Chlor-3-(1-pyrrolidino)-benzoyl-guanidin,
4-Methyl-3-(1-pyrrolidino)-benzoyl-guanidin,
4-Chlor-3-(1-piperidino)-benzoyl-guanidin,
4-Methyl-3-(1-piperidino)-benzoyl-guanidin,
4-Phenoxy-3-(1-pyrrolidino)-benzoyl-guanidin sowie
4-(2-Chlorphenoxy)-3-(1-pyrrolidino)-benzoyl-guanidin und deren pharmakologisch verträgliche Salze.

4. Verfahren zur Herstellung der Verbindungen I, nach Anspruch 1, dadurch gekennzeichnet, daß man
eine Verbindung der Formel II 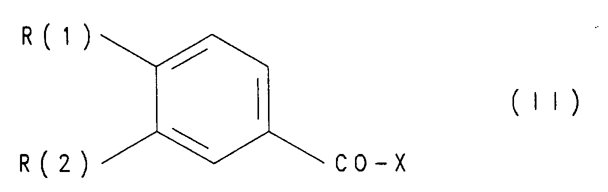 mit Guanidin der Formel III 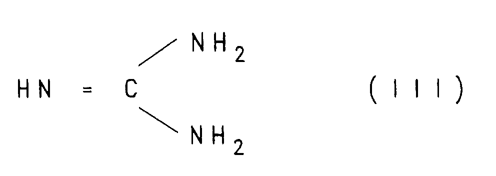 umsetzt, worin R(1) und R(2) die angegebene Bedeutung besitzen und X für eine leicht nucleophil substituierbare leaving group steht.

5. Verwendung einer Verbindung I nach Anspruch 1 zum Herstellen eines Medikaments zum Behandeln von Arrhythmien.

6. Verwendung einer Verbindung I nach Anspruch 1 zum Herstellen eines Medikaments zur Cardioprotektion.

## Claims

1. A benzoylguanidine of the formula I 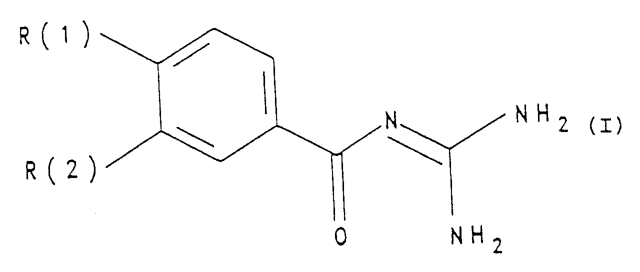 in which:
R(1) or R(2) is an amino group -NR(3)R(4),
where R(3) and R(4) - identical or different - are H, C₁-C₆-alkyl or C₃-C₇-cycloalkyl, or
R(3) is phenyl-(CH₂)ₚ- where p is 0, 1, 2, 3 or 4, or phenyl, where phenyl in each case is unsubstituted or carries one to two substituents from the groups fluorine, chlorine, methyl or methoxy,
R(3) and R(4) can also together be a straight-chain or branched C₄-C₇-methylene chain, where a -CH₂-member of the methylene chain can be replaced by oxygen, S or NR(5) and R(5) is H or lower alkyl,
the other substituent R(1) or R(2) in each case is F, Cl, C₁-C₄-alkyl, C₁-C₄-alkoxy, CF₃, CₘF₂ₘ₊₁-CH₂-, benzyl or phenoxy,
where the respective phenyl radical is unsubstituted or carries one to two substituents having the meaning methyl, methoxy, fluorine or chlorine,
and in which m is 1, 2 or 3,
or its pharmaceutically tolerable salts.

2. A benzoylguanidine of the formula I as claimed in claim 1, wherein:
one of the two substituents R(1) or R(2) is an amino group -NR(3)R(4) in which R(3) and R(4) together are a methylene chain (CH₂)ₙ where n = 4-5 and the other respective substituent R(1) or R(2) is chlorine or phenoxy.

3. A benzoylguanidine of the formula I as claimed in claim 1, which is:
4-chloro-3-(1-pyrrolidino)benzoylguanidine,
4-methyl-3-(1-pyrrolidino)benzoylguanidine,
4-chloro-3-(1-piperidino)benzoylguanidine,
4-methyl-3-(1-piperidino)benzoylguanidine,
4-phenoxy-3-(1-pyrrolidino)benzoylguanidine and
4-(2-chlorophenoxy)-3-(1-pyrrolidino)benzoylguanidine or a pharmacologically tolerable salt thereof.

4. A process for the preparation of the compounds I as claimed in claim 1, which comprises
reacting a compound of the formula II 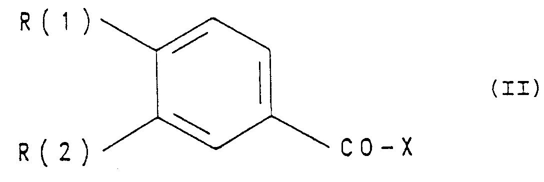 in which R(1) and R(2) have the given meaning and X is a leaving group which can be easily nucleophilically substituted,
with guanidine of the formula III

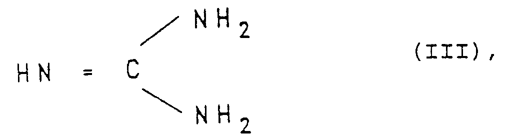

5. The use of a compound I as claimed in claim 1 for the production of a medicament for the treatment of arrhythmias.

6. The use of a compound I as claimed in claim 1 for the production of a medicament for cardioprotection.

## Revendications

1. Benzoylguanidines de formule I 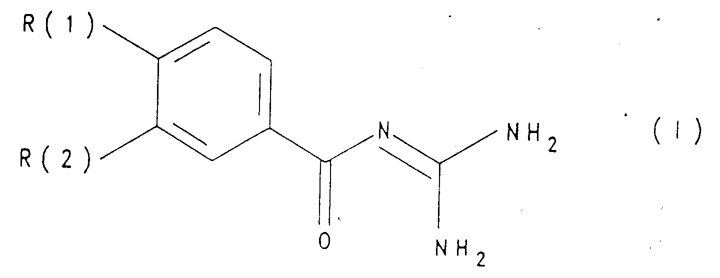 dans laquelle :
R(1) ou R(2) représentent un groupe amino -NR(3)R(4),
avec R(3) et R(4) - identiques ou différents - représentant un atome d'hydrogène, un radical alkyle en C₁-C₆, cycloalkyle en C₃-C₇, ou R(3) un groupe phényl(CH₂)ₚ - avec p égal à 0, 1, 2, 3 ou 4, ou un groupe phényle, le groupe phényle étant non substitué ou portant un ou deux substituants choisis parmi le fluor, chlore, méthyle ou méthoxy,
R(3) et R(4) pouvant aussi ensemble former une chaîne méthylène en C₄-C₇, linéaire ou ramifiée, un chaînon -CH₂- de la chaîne méthylène pouvant être substitué par un atome d'oxygène, de soufre ou un radical NR(5), R(5) représentant un atome d'hydrogène ou un radical alkyle inférieur,
l'autre substituant respectif R(1) ou R(2) représentant un atome de fluor, chlore, un groupe alkyle en C₁-C₄, alcoxy en C₁-C₄, CF₃, CₘF₂ₘ₊₁-CH₂-, benzyle ou phénoxy, le groupe phényle respectif étant non substitué ou portant un ou deux substituants choisis parmi des groupes méthyle, méthoxy, des atomes de fluor ou de chlore,
et m étant égal à 1,2 ou 3,
ainsi que leurs sels pharmaceutiquement acceptables.

2. Benzoylguanidines de formule I selon la revendication 1, caractérisées en ce que dans cette formule un des deux substituants R(1) ou R(2) représente un groupe amino -NR(3)R(4), formule dans laquelle R(3) et R(4) forment ensemble une chaîne méthylène (CH₂)ₙ avec n = 4 - 5 et l'autre substituant respectif R(1) ou R(2) est un atome de chlore ou un groupe phénoxy.

3. Benzoylguanidines de formule I selon la revendication 1, caractérisées en ce qu'elles sont :
la 4-chloro-3-(1-pyrrolidino)-benzoyl-guanidine,
la 4-méthyl-3-(1-pyrrolidino)-benzoyl-guanidine,
la 4-chloro-3-(1-pipéridino)-benzoyl-guanidine,
la 4-méthyl-3-(1-pipéridino)-benzoyl-guanidine,
la 4-phénoxy-3-(1-pyrroiidino)-benzoyl-guanidine ainsi que
la 4-(2-chlorophénoxy)-3-(1-pyrrolidino)-benzoyl-guanidine et leurs sels pharmacologiquement acceptables.

4. Procédé de préparation des composés I selon la revendication 1, caractérisé en ce que l'on fait réagir un composé de formule II 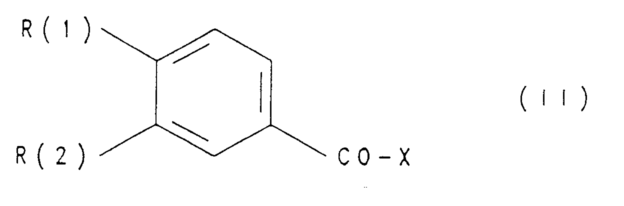 avec la guanidine de formule III 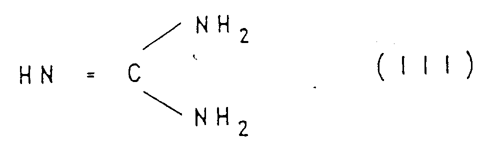 R(1) et R(2) ayant la signification indiquée et X représentant un groupe partant facilement substituable par un nucléophile.

5. Utilisation d'un composé 1 selon la revendication 1 pour la préparation d'un médicament pour le traitement des arythmies.

6. Utilisation d'un composé 1 selon la revendication 1 pour la préparation d'un médicament pour la protection cardiaque.
